# EUROPEAN PATENT SPECIFICATION

(11) **EP 2 438 882 B1**
(45) Date of publication and mention of the grant of the patent: **24.07.2019**
(21) Application number: 11183669.8
(22) Date of filing: 03.10.2011
(51) Int. Cl.: A61B 17/3207

(54) **Cutting wire assembly for use with a catheter**
Schnittdrahtanordnung zur Verwendung mit einem Katheter
Ensemble de découpe de fils pour une utilisation avec un cathéter

(30) Priority: 06.10.2010 US 390217 P; 18.11.2010 US 414931 P; 07.09.2011 US 201113226735; 07.09.2011 US 201113226699
(43) Date of publication of application: 11.04.2012
(73) Proprietor: Rex Medical, L.P., Conshohocken, PA 19428 (US); Schur, Israel, Teaneck, NJ 07666 (US)
(72) Inventor: Schur, Israel, Teaneck, New Jersey 07666 (US); Bressler, James Erich, Langhorne, Pennsylvania 19047 (US); McGuckin Jr., James F, Radnor, Pennsylvania 19087 (US)
(74) Representative: Lees, Kate Jane

(56) References cited:
- WO-A1-98/19608
- WO-A1-2010/011956
- WO-A2-2004/041329
- WO-A2-2010/003135
- US-A1- 2004 199 088
- US-A1- 2010 057 077

## Description

### Technical Field

This application relates to a system for treating stenotic lesions of a vessel and more particularly relates to a cutting wire for use with a catheter to open stenotic lesions in vessels.

### Background Art

Several methods have been utilized to treat stenotic lesions of vessels. With stenotic lesions, the vessel diameter is constricted and therefore attempts have been made to widen this constriction. One method is an invasive surgical procedure where the vessel wall is cut open and the portion containing the plaque or other constricting structure is removed. This procedure is traumatic, complex, and results in a long recovery time for the patient. It also causes a weakening of the vessel wall since a portion of the wall is removed. A weakened wall can ultimately result in an aneurysm which is a dilatation (expansion) of the artery, which adversely affects vessel function and if not surgically treated could be life threatening to the patient.

In order to reduce trauma to the patient, reduce the patient recovery time and reduce hospital costs, minimally invasive procedures have been developed to treat stenotic lesions. Balloon angioplasty is one such method. In angioplasty, a balloon is placed in the stenosed (restricted) portion of the vessel and inflated to compress the plaque against the vessel wall, thereby increasing the lumen in the vessel to improve blood flow. That is, the balloon is inflated to push the lesion radially outwardly to widen the passageway. Some stenotic lesions are resistant to conventional pressure balloons. Consequently, high pressure balloons have been developed to treat resistant stenotic lesions. However, such high pressure balloons apply more force and increase the risk of vessel trauma and rupture. Moreover, sometimes lesions are even resistant to these high pressure balloons.

Additionally, the use of these angioplasty balloon catheters oftentimes have only short term effect as it has been found that restensois frequently occurs after such treatment.

In an attempt to address such drawbacks as reducing the likelihood of restenosis and trauma, as well as to treat vessels with highly resistant lesions, cutting balloon catheters were developed. One such device is disclosed for example in U.S. Patent No. 5,196,024 which describes a catheter with a balloon and longitudinal cutting edges. One of the many disadvantages of this device, however, is it requires modifications of balloon catheters which significantly increases the cost of the catheter. Another disadvantage is that instead of using the procedural catheter, a different catheter may be required with a cutting balloon. Consequently, the surgeon would need to decide prior to the procedure which type of catheter to utilize, although this may not always be practical as the information to determine the type (e.g. resistance) of the lesion may not be available until the lesion is accessed and the extent of the disease is known. Thus, for example, the surgeon may insert an angioplasty catheter, inflate the balloon and find that it is insufficient to widen the vessel passageway. The surgeon would then need to conduct the time consuming task of removing the catheter and inserting a cutting balloon catheter, threading it through the vascular system over a guidewire. Since the catheters are inserted from a remote site, e.g. through the femoral artery, these catheter exchanges take time and increase trauma to the patient. Additionally, it adds to the cost of the procedure since two catheters would be required. In order to properly treat the diverse size and condition of each lesion a large inventory of multiple sized cutting balloons would be required.

Conversely, in certain procedures, utilizing a cutting balloon in soft lesions increases the risk of trauma or damage to the vessel and therefore it would not be desirable to use a cutting balloon catheter. Thus, an exchange for an angioplasty catheter would be necessary.

Such catheter exchanges might also require guidewire exchanges since the standard .035" (0.89 mm) guidewire utilized for an angioplasty catheter may be too large for the .018" (0.46 mm) cutting balloon catheter. The guidewire exchanges complicate the procedure, increase the risk to the patient and increase the procedure time, thereby increasing costs to the patient.

U.S. Patent No. 7,131,981 attempts to address the foregoing issues by providing a conversion device comprising an insertion tube insertable into the normal .035" (0.89 mm) guidewire lumen of an angioplasty catheter. This device would not work for angioplasty catheters with small guidewire lumens. The tube has two jacket segments and a guide insert device having a channel and four guide channels. Because of the complexity of the device, the cutting elements in the four channels would need to be sufficiently thin to be maintained in the smaller diameter device. Such thin (small diameter) cutting elements however may be too flexible and not have adequate stiffness to be effective. Additionally, the cutting elements are attached at one end, having an opposite free end which can potentially damage and perforate the vessel wall during use.

WO2010003135 describes an apparatus that is operable in different modes to perform various functions for treating a body lumen. The apparatus includes a shaft including a proximal end, a distal end, a lumen extending therebetween, and a balloon on the distal end having an interior communicating with the lumen. The apparatus includes a valve on the distal end that selectively opens or closes an outlet communicating with the lumen. With the valve open, fluid introduced into the lumen exits the outlet into a body lumen. With the valve closed, fluid introduced into the lumen expands the balloon. The apparatus also includes an actuator for axially compressing the balloon, and a helical member extends between ends of the balloon interior that expands the balloon from a contracted condition to an expanded helical shape when the actuator is activated.

The need therefore exists for an improved, more simplified device to enable the selective use of a cutting wire for treating stenosis.

### Summary of invention

The present invention overcomes the disadvantages and deficiencies of the prior art.

According to the present invention there is provided a device for treating a lesion in a body lumen to enlarge a passageway in the body lumen, the device comprising at least one cutting member having a proximal portion and a distal portion, and a tracking member having a proximal portion and a distal portion, the cutting member and tracking member being in the form of elongate members with a longitudinal axis of the cutting member extending alongside a longitudinal axis of the tracking member and being connected at their distal portions and insertable into the body lumen as a unit, wherein the cutting member is unattached to the tracking member at all points proximal of the distal connection between the cutting member and the tracking member and is configured for movement in a direction transverse to the longitudinal axis of the tracking member to widen a gap between the cutting member and tracking member proximal of the connection at the distal portions, and wherein the lengths of the tracking member and the cutting member are such as to extend in use out of the body for insertion of a catheter over the tracking member.

In some embodiments, the cutting member has a cutting surface on a first surface opposite a second surface facing the tracking member. The cutting surface may be formed only in a distal region of the cutting member. The second surface may have a convex surface.

In some embodiments, the tracking member includes a plurality of marker bands.

In some embodiments, the cutting member at a cutting region is substantially triangular in cross-section.

In some embodiments, only a distal region of the cutting member is provided with a cutting edge or surface, with the remaining proximal region thereof being atraumatic and non-cutting. A height of the distal region may be less than a height of the remaining proximal region.

In some embodiments, the tracking member has a coil at a distal tip.

In some embodiments, the device further comprises a shrink wrap over a connection region of the tracking and cutting members.

In some embodiments, the cutting member is a wire and the tracking member is a wire.

In some embodiments, the cutting member is substantially circular in cross-section in a proximal region and substantially triangular in cross-section in a distal region.

The device may be used in combination with a catheter having a lumen, wherein the at least one cutting member includes a plurality of cutting members, the plurality of cutting members are connected at a distal portion to the tracking member and insertable as a unit through the lumen of the catheter.

The invention also relates to a combination of a device as hereinbefore defined and a catheter having a lumen and an expandable portion, wherein the cutting member has a cutting surface configured to treat the lesion to enlarge the passageway in the body lumen when moved by the expandable portion of the catheter to widen the gap between the cutting member and tracking member. The expandable portion may comprise an inflatable balloon.

### Brief description of drawings

Preferred embodiment(s) of the present invention are described herein with reference to the drawings wherein:
Figure 1 is a perspective view of a conventional balloon catheter and a first embodiment of the cutting wire assembly (unit) of the present invention and showing the balloon expanded;
Figure 1A is a perspective view of the cutting assembly of Figure 1;
Figure 1B is a perspective view similar to Figure 1A showing the shrink wrap over a distal portion;
Figure 2A is an enlarged perspective view of the area of detail of Figure 1 showing a portion of the cutting wire in accordance with one embodiment;
Figure 2B is a perspective view similar to Figure 2A showing another embodiment of the cutting wire;
Figure 2C is a perspective view similar to Figure 2A showing yet another embodiment of the cutting wire;
Figure 2D is a perspective view similar to Figure 2A showing another embodiment of the cutting wire;
Figure 2E is a perspective view similar to Figure 2A of yet another embodiment of the cutting wire;
Figure 3A is a perspective view of another embodiment of the cutting wire assembly;
Figure 3B is a close up perspective view of a distal portion of the cutting assembly of Figure 3A.
Figure 4A is a cross-sectional view of the cutting wire taken along line 4A-4A of Figure 3B;
Figure 4B is a cross-sectional view of the cutting wire taken along line 4B-4B of Figure 3B;
Figures 5-5E illustrate the method steps for use of the cutting wire assembly of Figure 1, the drawings showing cross-sectional views, wherein

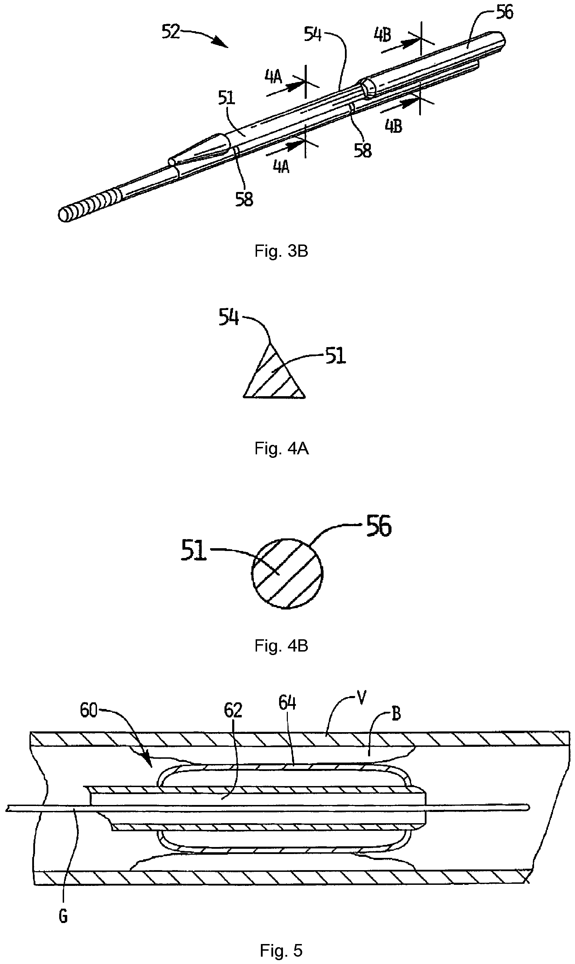

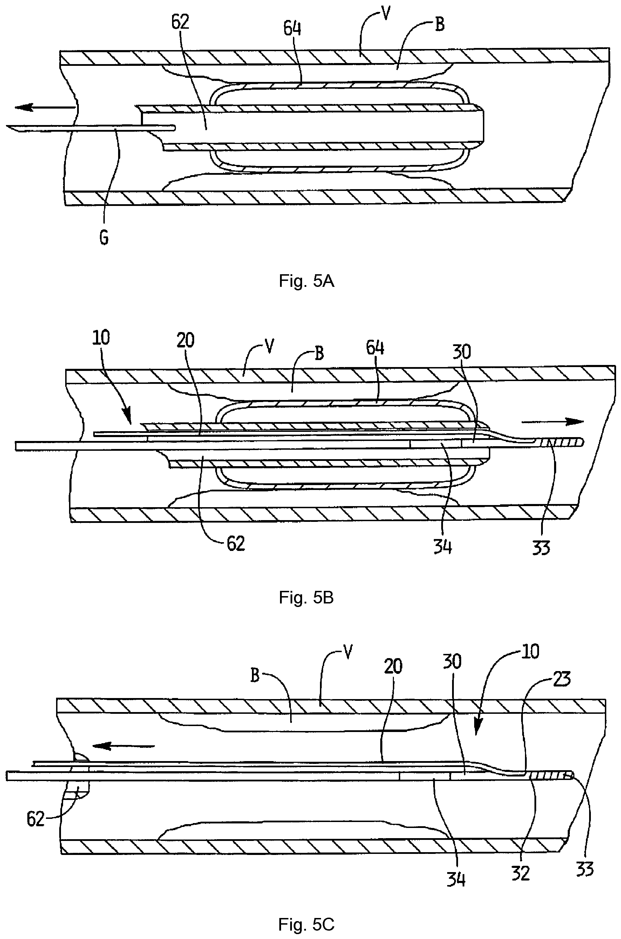

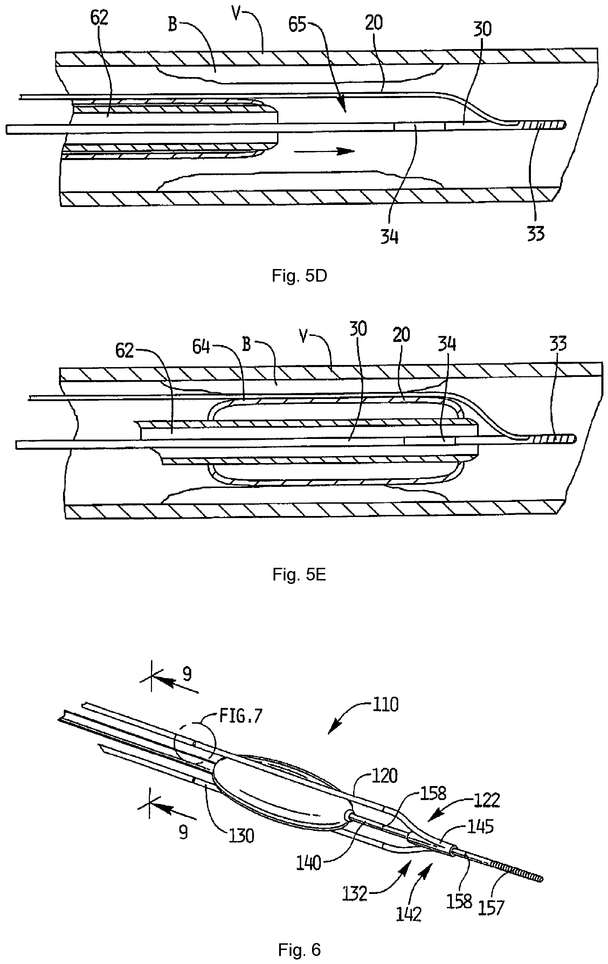

Figure 5 illustrates a conventional balloon catheter inserted over a conventional guidewire;
Figure 5A illustrates withdrawal of the conventional guidewire;
Figure 5B illustrates insertion of the cutting and tracking members of the present invention through the balloon catheter lumen;
Figure 5C illustrates withdrawal of the balloon catheter leaving the cutting and tracking members within the vessel lumen;
Figure 5D illustrates the balloon catheter inserted over the tracking member; and
Figure 5E illustrates expansion of the balloon of the balloon catheter to force the cutting wire into cutting contact with the lesion.

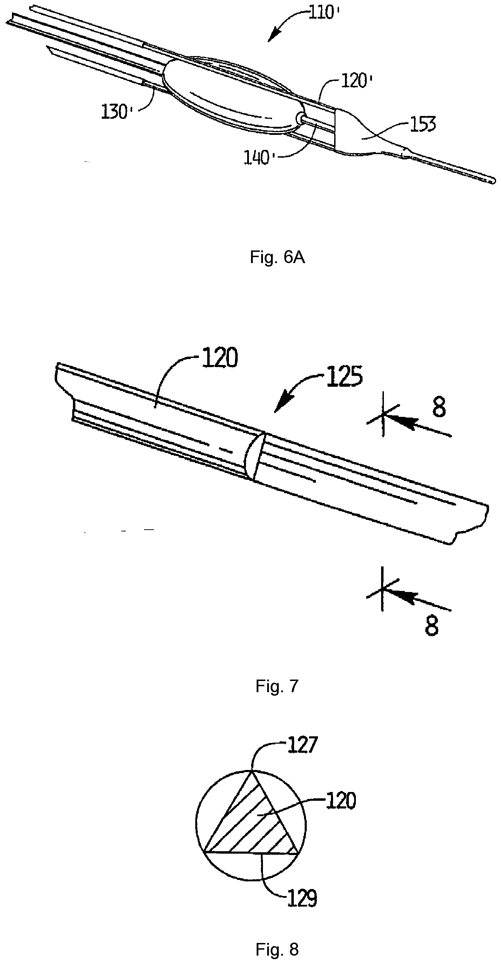

Figure 6 is a perspective view of a conventional balloon catheter and an alternate embodiment of the cutting wire assembly (unit) of the present invention, and showing the balloon expanded;
Figure 6A is perspective view of an alternate embodiment of the cutting wire assembly;
Figure 7 is a perspective view of the area of detail of Figure 6 showing a portion of the cutting wire in accordance with one embodiment;

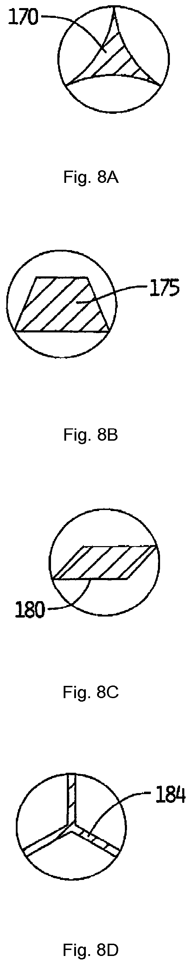

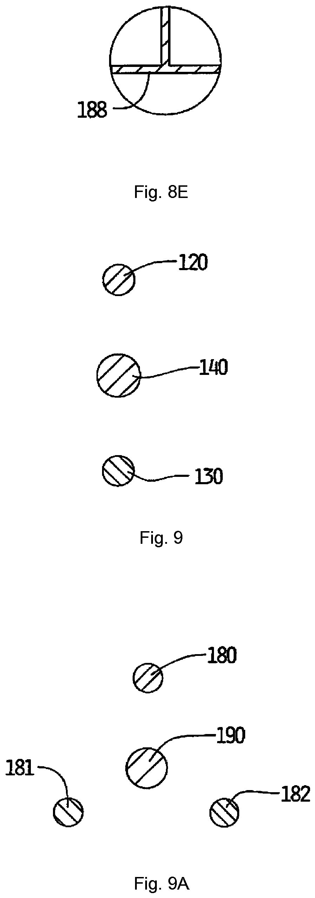

Figure 8 is a cross-sectional view of the cutting wire taken along line 8-8 of Figure 7;
Figures 8A-8E are views similar to Figure 8 showing cross-sectional views of alternate embodiments of the cutting wire of the present invention;
Figure 9 is a cross-sectional view taken along line 9-9 of Figure 6;
Figure 9A is a cross-sectional view of another embodiment of the cutting wire assembly of the present invention;

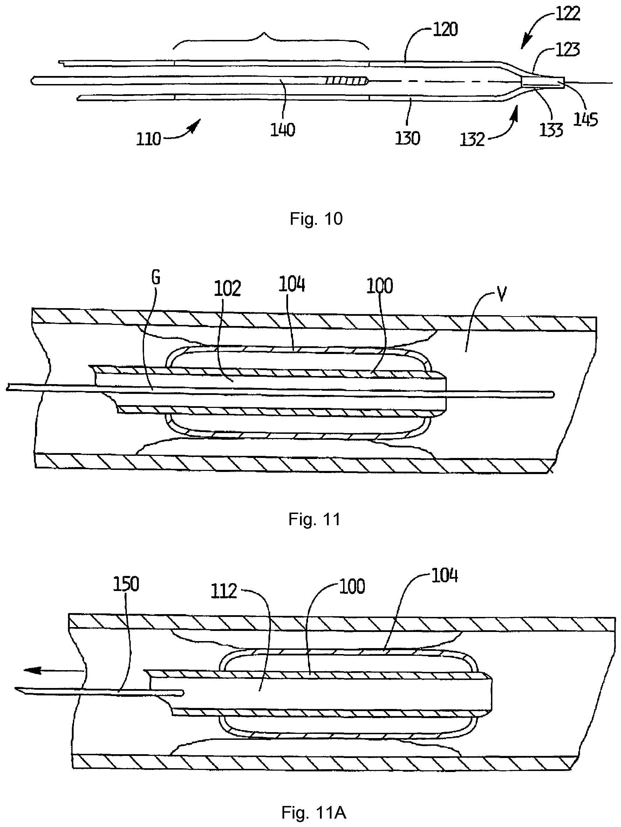

Figure 10 is a partially exploded side view of the cutting wire assembly of Figure 6;

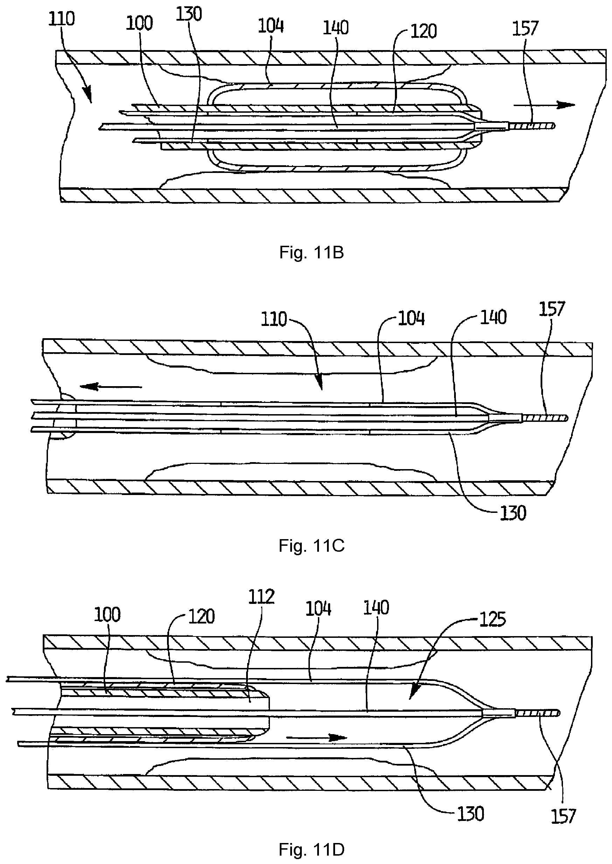

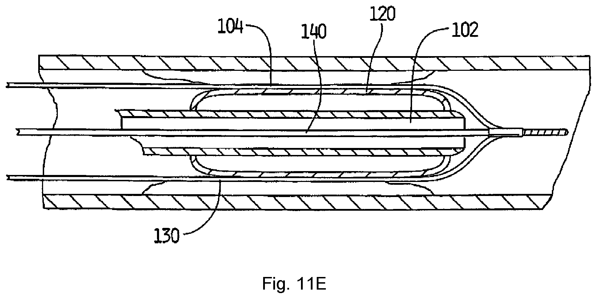

Figures 11-11E illustrate the method steps for use of the cutting wire assembly of Figure 6, the drawings showing cross-sectional views, wherein:
   Figure 11 illustrates a conventional balloon catheter inserted over a conventional guidewire;
   Figure 11A illustrates withdrawal of the conventional guidewire;
   Figure 11B illustrates insertion of the cutting and tracking elements of the present invention through the balloon catheter lumen;
   Figure 11C illustrates withdrawal of the balloon catheter;
   Figure 11D illustrates the balloon catheter inserted over the tracking element; and
   Figure 11E illustrates expansion of the balloon of the balloon catheter to force the cutting elements into cutting contact with the lesion.

### Description of embodiments

Referring now in detail to the drawings wherein like reference numerals identify similar or like components throughout the several views, the cutting assembly of the present invention includes a cutting member (or element) and a tracking member (or element). In some embodiments, a single cutting member is provided; in alternative embodiments, multiple cutting members are provided.

The various devices of the present invention disclosed herein function to treat the stenotic lesion inside the vessel wall, thereby opening or enlarging the passageway in the vessel which was restricted. The stenosis can be a result of plaque buildup, endothelial growth, blood clots, etc. The device can also be used to treat other lesions restricting passageways in other body lumens.

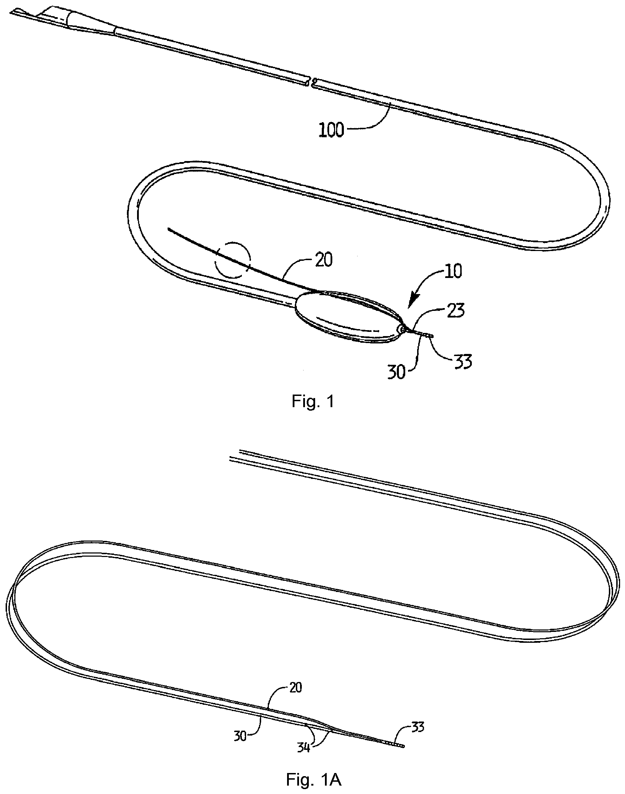

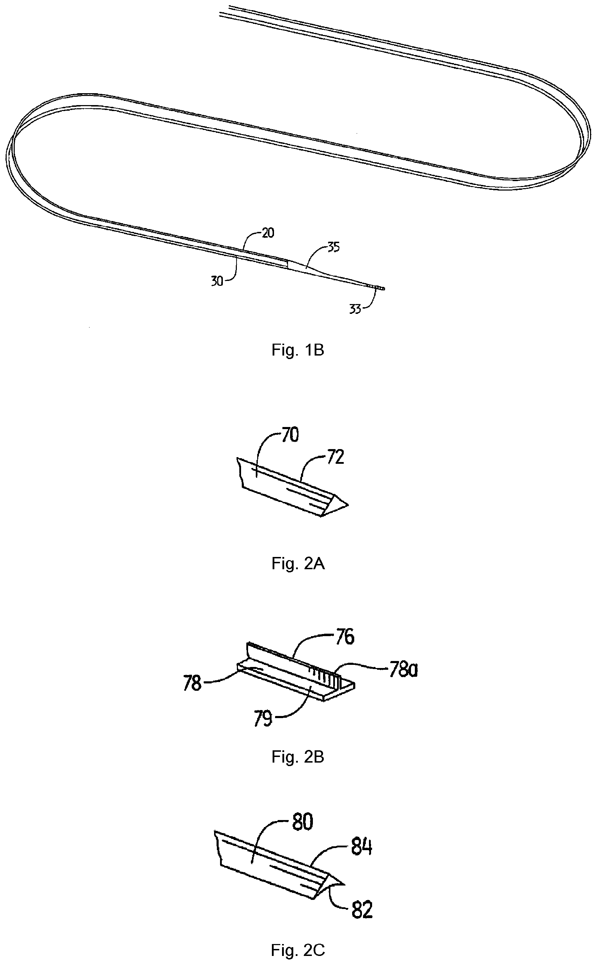

With initial reference to the single cutting member embodiments of Figures 1-5, an assembly 10 in accordance with one embodiment of the present invention includes a tracking member, designated generally by reference numeral 30 and preferably in the form of a wire, and a cutting member, designated generally by reference numeral 20 and preferably in the form of a wire. As discussed in more detail below, the tracking wire 30 and cutting wire 20 are preferably attached at a distal portion so they are insertable as a unit. The wire assembly 10, as described below, can be used with a conventional catheter, such as an angioplasty catheter.

With reference to Figures 1 and 5C, cutting member in the form of a wire 20 has a distal portion 23 which is connected to a distal portion 32 of a tracking member in the form of a wire 30. In the illustrated embodiment, a distalmost tip 23 of the cutting wire 20 is attached to the tracking or guidewire 30. One way of attachment is to twist the wires together. Other methods of attachment are also contemplated such as welding, bonding or placement of a separate element such as a collar over the end of the wires to frictionally engage the wires. The cutting wire 20 remains unattached proximal of the distal connection (attachment) region to enable it to be separated from the tracking wire 30, e.g. moved transversely with respect to the longitudinal axis of the tracking wire 30. In Figure 5C, the initial position of the wires 20, 30 are shown; in Figures 5D and 5E the wires 20, 30 are further separated as described in detail below.

Tracking member has a coil tip 33 for flexibility. A heat shrink wrap 35 can be placed over the connection region of the cutting wire 20 and a tracking wire 30. Marker bands 34 can be provided for imaging.

Note the tracking wire and cutting wire can be of substantially the same length, both extending out of the body for reinsertion of a catheter over the tracking wire as described below. Alternatively, they can be of different lengths.

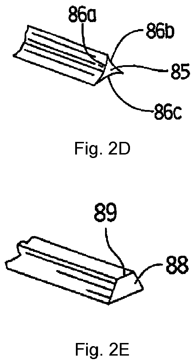

Various configurations of the cutting wire 20 are illustrated to effectively treat lesions. In the embodiment of Figure 2A, the wire 70 is substantially triangular in cross section forming a V-shaped cutting surface 72 on a first surface opposite a second surface facing the tracking wire 30. In the embodiment of Figure 2B, a cutting edge 76 on first surface 78a of cutting wire 78 with a substantially planar outer edge extends from the substantially planar base 79. In Figure 2C, a surface 82 of the wire 80 opposite the cutting surface 84 and facing the tracking wire 30 is convex. This surface 82 can conform to the outer surface of the catheter balloon. Two or alternatively three sides of the cutting wire can be convex as in sides 86a, 86b, 86c of wire 85 of Figure 2D. In the embodiment of Figure 2E, wire 88 is trapezoidal in cross section with a cutting surface 89 on the outer surface. Other shapes are also contemplated, including but not limited to polygonal shapes that are substantially: square, rectangular, rhombus, hexagonal, pentagonal, octagonal, diamond shaped, etc. A round or oval wire cross-section with a sharpened surface is also contemplated. Caltrop shapes and upside down T-shapes are also contemplated. These wire shapes can be utilized in the various cutting wire embodiments disclosed herein.

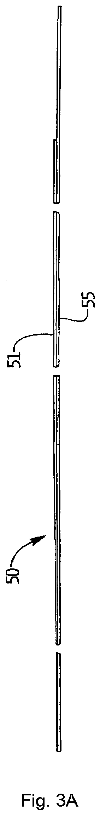

Note, if desired, only a portion of the cutting wire (member) can have the cutting edge or surface, e.g. the distal region, with a remaining portion being atraumatic and non-cutting. This is shown for example in the embodiment of Figures 3A and 3B where wire 51 of cutting assembly 50 has a distal portion having a cutting edge 54. (Although shown as a substantially triangular cross-section, other cross-sectional shapes are also contemplated including those discussed above). Proximal of distal portion 52, the portion 56 is atraumatic and can be substantially circular in cross-section as shown in Figure 4B. (Other shapes are also contemplated). The distal region of wire 51 can be conical and can have an atraumatic surface. Note the height of the cutting surface region can be less than the height (e.g. diameter) of the atraumatic portion 56.

A coil such as coil 33 can be provided at the distal tip of tracking member (wire) 55. Several marker bands 58 are provided on tracking wire 55 for imaging. A heat shrink wrap such as shrink wrap 35 of Figure 1B which covers the distal and connecting region of wires 20 and 30 can be provided over the distal tip and connection region of the cutting and tracking wires 51, 55 of Figure 3B.

One method of use of the wire assembly 10 of the present invention will now be described. The method is described for using wire assembly 10, but it should be appreciated that the same method can be used for wire assembly 50 (and for the other cutting wire configurations). Initially, a conventional catheter 60, such as conventional angioplasty catheter, is inserted over a conventional guidewire G to the treatment site as shown in Figure 5. Guidewire G extends through a lumen 62 in the catheter 60. Access to the vessel can be obtained through the femoral artery or vein for example. Note the proximal end of the catheter 60 and guidewire G extend outside the patient's body. The angioplasty catheter 60 has an inflatable balloon 64 which is in fluid communication with an inflation lumen of the catheter as is conventional. At the target site, inflation of the balloon 64 expands the balloon 64 to expand the lesion B and widen the lumen of the vessel V.

If the stenotic lesion cannot be successfully opened by a conventional balloon due to lack of force, the wire assembly 10 (or assembly 50) of the present invention can be utilized. In this case, the guidewire G is removed from the guidewire lumen 62 of the catheter 60 (see Figure 5A) and the wire assembly 10 (or 50) is inserted through the lumen 62 as shown in Figure 5B. Thus, by insertion through the lumen 62, the tracking guidewire 30 (or 55) and cutting wire 20 (or 51) of wire assembly 10 (or 50) are inserted to the target site.

Next, the catheter 60 is removed from the treatment site and vessel, and removed from the body, leaving the wire assembly 10 at the target site as shown in Figure 5C. The catheter 60 is then reinserted over proximal end of tracking wire 30. Note that instead of reinserting the same catheter used in the step of Figure 5, alternatively, a different balloon catheter (or catheter with other expandable member) can be inserted. In either event, the catheter is inserted over the proximal portion of the tracking wire 30 such that the tracking wire 30 extends through the lumen 62; however, cutting wire 20 remains outside the lumen 62 and thus does not extend through lumen 62 as shown in Figure 5D. In this manner, the tracking wire 30 provides a guide for the catheter 60 to the target site, while the cutting wire 20 remains adjacent an outer surface of the catheter 60 for subsequent expansion into contact with the lesion. As shown in Figure 5D, there is an increased gap 65 between the cutting wire 20 and tracking wire 30 caused by the catheter 60 positioned between the two wires 20, 30.

To expand or move the wire 20 transversely with respect to the longitudinal axis of the tracking wire 30 (and transverse to the longitudinal axis of the catheter 60), the balloon 64 is inflated, forcing the cutting wire 20 radially and into contact with the lesion B so the cutting edge or surface can treat the lesion. It should be appreciated that instead of a balloon, a mechanical expander or other structure can be used to force the cutting wire 20 into contact with the lesion. If desired, the balloon 64 can be deflated and the wire assembly easily rotated to another position for subsequent transverse movement by the cutting wire into contact with another region of the lesion B. In this manner, the select portions of the stenosis can be treated, as the cutting wire 20 is expanding in one direction. The cutting wire assembly 50 can be used in a similar manner.

As can be appreciated, the method described above utilizes the same catheter for the initial step (Figure 5) as well as for the subsequent step of reinsertion for placement only over the tracking wire 30 (Figure 5D). However, it is also contemplated that a different catheter can be used for insertion over tracking wire 30 in the step of Figure 5D.

Alternative embodiments having multiple cutting wires are illustrated in Figures 6-11. More specifically, cutting assembly 110 of one embodiment includes a tracking member, designated generally by reference numeral 140, preferably in the form of a wire, and two cutting members, designated generally by reference numerals 120 and 130, and preferably in the form of wires. As discussed in more detail below, the tracking wire 140 and cutting wires 120, 130 are preferably attached at a distal portion so they are insertable as a unit. The wire assembly 110, as described below, can be used with a conventional catheter, such as an angioplasty catheter. Additionally, although two cutting wires are shown in Figure 6, spaced about 180 degrees apart, different spacing is also contemplated. Additionally, more than two cutting wires can be provided, e.g. three cutting wires such as wires 180, 181, 182 of Figure 9A, four cutting wires, etc. The three cutting wires 180, 181, 182, can be equidistantly spaced apart encircling tracking wire 190 as shown or spaced at different distances.

With reference to Figures 6, 10 and 11C, the first cutting member is in the form of a wire 120 and has a distal portion 122 which is connected to a distal portion 142 of a tracking member in the form of a wire 140. In the illustrated embodiment, a distalmost tip 123 of the cutting wire 120 is attached to the tracking guidewire 140. Similarly, the second cutting member is in the form of a wire 130 and has a distal portion 132 which is connected to the distal portion 142 of the tracking member 140. In the illustrated embodiment, a distalmost tip 133 of the cutting wire 130 is attached to the tracking guidewire 140. One way of attachment of the wires 120, 130 to wire 140 is to twist the wires together. Other methods of attachment are also contemplated such as welding, bonding or placement of a separate element such as a collar, e.g. collar 145, over the end of the wires to frictionally engage the wires. The cutting wires 120, 130 remain unattached proximal of the distal connection to enable them to be separated from the tracking wire 140, e.g. moved transversely with respect to the longitudinal axis of the tracking wire 140. In Figures 11B and 11C, the initial position of the wires are shown; in Figures 11D and 11E the wires are separated as described in detail below.

A coil 157 can be provided at the tip such as illustrated in Figure 6. Several marker bands 158 can be provided on tracking wire 140 for imaging. In the alternate embodiment of Figure 6A, a shrink wrap 153 can be provided over the connection region of the tracking wire 140' and cutting wires 120', 130' of cutting assembly 110'. The cutting assembly 110' can also include a coil similar to coil 157 and a shrink wrap similar to shrink wrap 153.

Note the tracking wire and cutting wires can be of substantially the same length, both extending out of the body for reinsertion of a catheter over the tracking wire as described below. Alternatively, they can be of different lengths.

Various configurations of the cutting wires 120, 130 are illustrated to effectively treat lesions. In the embodiment of Figure 6, the wires 120, 130 are substantially circular in cross-section until a transition region, i.e. region 125, where it transitions to a wire substantially triangular in cross section forming a V-shaped cutting surface 127 on a first surface opposite a second surface 129 facing the tracking wire 140 (Figure 8). A concave or convex surface can be formed on one, two or all three sides (see e.g. wire 170 of Figure 8A). A convex surface on the side opposite the cutting edge helps to conform to the outer surface of the catheter balloon.

Other cross-sectional shapes of the cutting wires 120, 130 are also contemplated, including but not limited to, polygonal shapes that are substantially: rectangular, square, trapezoidal (see e.g. wire 175 of Figure 8B), hexagonal, pentagonal, octagonal, diamond shaped, etc. A round or oval wire cross-section with a sharpened surface is also contemplated. In the embodiment of Figure 8C, a rhombus shaped wire 180 is illustrated. This shape facilitates cutting if the cutting wire is rotated. Figure 8D illustrates a caltrop shape wire 184 configured so that one point will always point upward. Figure 8E illustrates an upside down T-shape wire 188. The base of wire 188 can be convex. These wire shapes can be utilized in the various cutting wire embodiments disclosed herein.

It is contemplated that cutting wire 130 has the same configuration as cutting wire 120. However, in alternate embodiments, the cutting wire 130 can have an alternate configuration, including but not limited to, any of the foregoing cross-sectional wire shapes.

Note, if desired, only a portion of the cutting wires 120, 130 can have the cutting edge or surface, e.g. the distal region, with a remaining portion being atraumatic and non-cutting. This is illustrated in Figure 7, where the circular cross-section at a more proximal region is atraumatic. Note a region distal of the cutting portion can be atraumatic, e.g. can transition back to a substantially circular cross-section. The wires can also have a conical tip as in the embodiment of Figure 3B.

One method of use of the wire assembly 110 of the present invention will now be described. Wire assembly 110' would be used in the same manner. Initially, a conventional angioplasty catheter 100 is inserted over a conventional guidewire G to the treatment site as shown in Figure 11. Guidewire G extends through a lumen 102 in the catheter 100. Access to the vessel can be obtained through the femoral artery or vein for example. Note the proximal end of the catheter 100 and guidewire G extend outside the patient's body. The angioplasty catheter 100 has an inflatable balloon 104 which is in fluid communication with an inflation lumen of the catheter as is conventional. At the target site, inflation of the balloon 104 expands the balloon to expand the lesion B and widen the lumen of the vessel V.

If the stenotic lesion cannot be successfully opened by a conventional balloon due to lack of force, the wire assembly 110 (or 110') of the present invention can be utilized. In this case, the guidewire G is removed from the guidewire lumen 102 of the catheter 100 (see Figure 11B) and the wire assembly 110 is inserted through the lumen 102 as shown in Figure 11B. Thus, by insertion through the lumen 102, the tracking guidewire 140 and cutting wires 120, 130 of wire assembly 110 are inserted to the target site.

Next, the catheter 100 is removed from the treatment site and vessel, and removed from the body, leaving the wire assembly 110 at the target site as shown in Figure 11C. The catheter 100 is then reinserted over the proximal end of tracking wire 140. Note that instead of reinserting the same catheter used in the step of Figure 11, alternatively, a different balloon catheter can be inserted. In either event, the catheter 100 is inserted over the proximal portion of the tracking wire 140 such that the tracking wire 140 extends through the lumen 102; however, cutting wires 120 and 130 remain outside the lumen 102 as shown in Figure 11D. In this manner, the tracking wire 140 provides a guide for the catheter 100 to the target site, while the cutting wires remain adjacent an outer surface of the catheter 100 for subsequent expansion into contact with the lesion. As shown in Figure 11D, there is an increased gap 125 between the cutting wires 120, 130 and tracking wire 140 caused by the catheter 100 positioned between the tracking wire 140 and the two wires 120, 130.

To expand or move the cutting wires 120, 130 transversely with respect to the longitudinal axis of the tracking wire 140 (and transverse to the longitudinal axis of the catheter 100), the balloon 104 is inflated, forcing the cutting wires 120 and 130 into contact with the lesion B so the cutting edge or surface can treat the lesion. It should be appreciated that instead of a balloon, a mechanical expander or other structure can be used to force the cutting wires 120, 130 radially and into contact with the lesion. If desired, the balloon 104 can be deflated and the wire assembly easily rotated to another position for subsequent transverse movement of the cutting wires by the balloon into contact with another region of the lesion B to treat select portions of the stenosis.

As can be appreciated, the method described above utilizes the same catheter for the initial step (Figure 11) as well as for the subsequent step of reinsertion for placement only over the tracking wire 140 (Figure 11D). However, it is also contemplated that a different catheter can be used for reinsertion over only the tracking wire 140.

Also, as shown, a single balloon is utilized to expand both wires substantially simultaneously. It is also contemplated that a separate balloon or separate expansion of the single balloon can be used to move the wires 120, 130 independently/separately.

As can be appreciated, the wire assemblies disclosed herein can accommodate balloon catheters having relatively small guidewire lumens.

Also, although access is described through the femoral artery, other approaches to the target site are also contemplated. Additionally, although described for use to treat lesions in vessel lumens, the devices disclosed herein can also be used to remove other structures constricting the passageway in the vessel or in other body lumens.

The cutting and tracking components are illustrated as wires, but other structures for the cutting member and tracking member are also contemplated such as a hard plastic tube or a metal hypotube. The metal hypotube can be formed with a cutting surface or alternatively have a cutting member such as a cutting tube attached thereto.

The cutting wire assemblies of the present invention as described can be used in various vessels including for example, veins such as the femoral veins, grafts such as dialysis grafts, etc. Other vessels are also contemplated such as use in carotid arteries, coronary arteries, the descending aorta and renal arteries, the external iliac and internal iliac arteries and the common femoral and deep femoral arteries. Applications for the devices disclosed herein include, but are not limited to, treating stenotic venous and arterial anastomosis, lesions resistant to conventional angioplasty, stent restenosis, and vessels with buildup of intima, etc.

While the above description contains many specifics, those specifics should not be construed as limitations on the scope of the disclosure, but merely as exemplifications of preferred embodiments thereof. Those skilled in the art will envision many other possible variations that are within the scope of the disclosure as defined by the claims appended hereto.

## Claims

1. A device for treating a lesion in a body lumen to enlarge a passageway in the body lumen, the device comprising at least one cutting member (20, 120, 120', 130', 130) having a proximal portion and a distal portion (23, 122, 132), and a tracking member (30,140) having a proximal portion and a distal portion (142), the cutting member and tracking member being in the form of elongate members with a longitudinal axis of the cutting member extending alongside a longitudinal axis of the tracking member and being connected at their distal portions and insertable into the body lumen as a unit, **characterised in that** the cutting member is unattached to the tracking member (30, 140) at all points proximal of the distal connection between the cutting member and the tracking member and is configured for movement in a direction transverse to the longitudinal axis of the tracking member to widen a gap between the cutting member and tracking member proximal of the connection at the distal portions, and **in that** the lengths of the tracking member and the cutting member are such as to extend in use out of the body for insertion of a catheter over the tracking member.

2. The device of claim 1, wherein the cutting member (20, 120) has a cutting surface (72, 127) on a first surface opposite a second surface (129) facing the tracking member (30, 140).

3. The device of claim 2, wherein the cutting surface is formed only in a distal region of the cutting member.

4. The device of claim 2 or 3, wherein the second surface has a convex surface.

5. The device of any preceding claim, wherein the tracking member (30, 140) includes a plurality of marker bands (34, 58, 158).

6. The device of any preceding claim, wherein the cutting member (20) at a cutting region (70, 80, 85) is substantially triangular in cross-section.

7. The device of any preceding claim, wherein only a distal region of the cutting member (20) is provided with a cutting edge or cutting surface (54), with the remaining proximal region (56) thereof being atraumatic and non-cutting.

8. The device of claim 7, wherein a height of the distal region is less than a height of the proximal region.

9. The device of any preceding claim, wherein the tracking member (30, 140) has a coil (33, 157) at a distal tip.

10. The device of any preceding claim, further comprising a shrink wrap (35, 153) over a connection region of the tracking and cutting members (30, 140', 20, 120', 130').

11. The device of any preceding claim, wherein the cutting member (20, 120, 130) is a wire (51, 70, 170, 175, 180, 181, 182, 184, 188) and the tracking member (30, 140) is a wire (55).

12. The device of any preceding claim, wherein the cutting member (20, 120, 130) is substantially circular in cross-section in a proximal region (56) and substantially triangular in cross-section in a distal region (51).

13. The combination of a device of any preceding claim with a catheter (100) having a lumen, wherein the at least one cutting member includes a plurality of cutting members (120, 130), the plurality of cutting members are connected at a distal portion (142) to the tracking member (140) and insertable as a unit through the lumen of the catheter (100).

14. The combination of the device of any one of claims 1 to 12 with a catheter (60, 100) having a lumen (62,102) and an expandable portion (64, 104), wherein the cutting member (20, 120, 120', 130, 130') has a cutting surface configured to treat the lesion to enlarge the passageway in the body lumen when moved by the expandable portion of the catheter to widen the gap between the cutting member and tracking member (30, 140).

15. The combination of claim 14, wherein the expandable portion (64, 104) comprises an inflatable balloon.

## Patentansprüche

1. Vorrichtung für die Behandlung einer Läsion in einem Körperlumen, um einen Durchgang in dem Körperlumen zu vergrößern, wobei die Vorrichtung Folgendes umfasst: mindestens ein Schneideelement (20, 120, 120', 130', 130), das einen proximalen Teil und einen distalen Teil (23, 122, 132) aufweist, und ein Führungselement (30, 140), das einen proximalen Teil und einen distalen Teil (142) aufweist, wobei das Schneideelement und Führungselement in Form von langgestreckten Elementen vorliegen, wobei sich eine Längsachse des Schneideelements entlang einer Längsachse des Führungselements erstreckt, und an ihren distalen Teilen verbunden sind und in das Körperlumen als eine Einheit einführbar sind, **dadurch gekennzeichnet, dass** das Schneideelement an dem Führungselement (30, 140) an allen Punkten ungebunden ist, die sich proximal von der distalen Verbindung zwischen dem Schneideelement und dem Führungselement befinden, und für eine Bewegung in einer Richtung konfiguriert ist, die quer zu der Längsachse des Führungselements verläuft, um einen Zwischenraum zwischen dem Schneideelement und Führungselement proximal von der Verbindung an den distalen Teilen auszuweiten, und dadurch, dass die Längen des Führungselements und des Schneideelements derart sind, dass sie sich bei Verwendung aus dem Körper für die Einführung eines Katheters über das Führungselement erstrecken.

2. Vorrichtung nach Anspruch 1, wobei das Schneideelement (20, 120) eine Schneideoberfläche (72, 127) auf einer ersten Oberfläche aufweist, die einer zweiten Oberfläche (129) gegenüberliegt, die dem Führungselement (30, 140) zugewandt ist.

3. Vorrichtung nach Anspruch 2, wobei die Schneideoberfläche nur in einem distalen Bereich des Schneideelements ausgebildet ist.

4. Vorrichtung nach Anspruch 2 oder 3, wobei die zweite Oberfläche eine konvexe Oberfläche aufweist.

5. Vorrichtung nach einem vorstehenden Anspruch, wobei das Führungselement (30, 140) eine Vielzahl von Markierungsbändern (34, 58, 158) einschließt.

6. Vorrichtung nach einem vorstehenden Anspruch, wobei das Schneideelement (20) an einem Schneidebereich (70, 80, 85) im Querschnitt im Wesentlichen dreieckig ist.

7. Vorrichtung nach einem vorstehenden Anspruch, wobei nur ein distaler Bereich des Schneideelements (20) mit einer Schneidkante oder Schneideoberfläche (54) bereitgestellt ist, wobei der restliche proximale Bereich (56) davon atraumatisch und nicht schneidend ist.

8. Vorrichtung nach Anspruch 7, wobei eine Höhe des distalen Bereichs kleiner ist als eine Höhe des proximalen Bereichs.

9. Vorrichtung nach einem vorstehenden Anspruch, wobei das Führungselement (30, 140) eine Spirale (33, 157) an einer distalen Spitze aufweist.

10. Vorrichtung nach einem vorstehenden Anspruch, die weiter eine Schrumpffolie (35, 153) über einem Verbindungsbereich des Führungs- und Schneideelements (30, 140', 20, 120', 130') umfasst.

11. Vorrichtung nach einem vorstehenden Anspruch, wobei das Schneideelement (20, 120, 130) ein Draht (51, 70, 170, 175, 180, 181, 182, 184, 188) ist und das Führungselement (30, 140) ein Draht (55) ist.

12. Vorrichtung nach einem vorstehenden Anspruch, wobei das Schneideelement (20, 120, 130) in einem proximalen Bereich (56) im Querschnitt im Wesentlichen kreisförmig und in einem distalen Bereich (51) im Querschnitt im Wesentlichen dreieckig ist.

13. Kombination einer Vorrichtung nach einem vorstehenden Anspruch mit einem Katheter (100), der ein Lumen aufweist, wobei das mindestens eine Schneideelement eine Vielzahl von Schneideelementen (120, 130) einschließt, die Vielzahl von Schneideelementen an einem distalen Teil (142) mit dem Führungselement (140) verbunden und als eine Einheit durch das Lumen des Katheters (100) einführbar ist.

14. Kombination der Vorrichtung nach einem der Ansprüche 1 bis 12 mit einem Katheter (60, 100), der ein Lumen (62, 102) und einen ausdehnbaren Teil (64, 104) aufweist, wobei das Schneideelement (20, 120, 120', 130, 130') eine Schneideoberfläche aufweist, die zur Behandlung der Läsion konfiguriert ist, um den Durchgang in dem Körperlumen zu vergrößern, wenn es von dem ausdehnbaren Teil des Katheters bewegt wird, um den Zwischenraum zwischen dem Schneideelement und Führungselement (30, 140) auszuweiten.

15. Kombination nach Anspruch 14, wobei der ausdehnbare Teil (64, 104) einen aufblasbaren Ballon umfasst.

## Revendications

1. Dispositif destiné à traiter une lésion dans une lumière corporelle pour agrandir un passage dans la lumière corporelle, le dispositif comprenant au moins un élément de coupe (20, 120, 120', 130', 130) présentant une partie proximale et une partie distale (23, 122, 132), et un élément de suivi (30, 140) présentant une partie proximale et une partie distale (142), l'élément de coupe et l'élément de suivi se présentant sous forme d'éléments allongés avec un axe longitudinal de l'élément de coupe s'étendant le long d'un axe longitudinal de l'élément de suivi et étant connectés au niveau de leurs parties distales et insérables dans la lumière corporelle en tant que tout, **caractérisé en ce que** l'élément de coupe n'est pas attaché à l'élément de suivi (30, 140) à tous les points de la connexion distale entre l'élément de coupe et l'élément de suivi et est configuré pour se déplacer dans un sens transversal à un axe longitudinal de l'élément de suivi pour élargir un espace entre l'élément de coupe et l'élément de suivi proximal à la connexion au niveau des parties distales, et **en ce que** les longueurs de l'élément de suivi et de l'élément de coupe sont telles qu'elles s'étendent en service en dehors du corps pour l'insertion d'un cathéter par-dessus l'élément de suivi.

2. Dispositif selon la revendication 1, dans lequel l'élément de coupe (20, 120) présente une surface de coupe (72, 127) sur une première surface opposée à une seconde surface (129) faisant face à l'élément de suivi (30, 140).

3. Dispositif selon la revendication 2, dans lequel la surface de coupe est formée uniquement dans une région distale de l'élément de coupe.

4. Dispositif selon la revendication 2 ou 3, dans lequel la seconde surface présente une surface convexe.

5. Dispositif selon n'importe quelle revendication précédente, dans lequel l'élément de suivi (30, 140) comporte une pluralité de bandes repères (34, 58, 158).

6. Dispositif selon n'importe quelle revendication précédente, dans lequel l'élément de coupe (20) au niveau d'une région de coupe (70, 80, 85) présente une coupe transversale sensiblement triangulaire.

7. Dispositif selon n'importe quelle revendication précédente, dans lequel seule une région distale de l'élément de coupe (20) est pourvue d'un bord de coupe ou d'une surface de coupe (54), la région proximale restante (56) de celui-ci étant atraumatique et non coupante.

8. Dispositif selon la revendication 7, dans lequel une hauteur de la région distale est inférieure à une hauteur de la région proximale.

9. Dispositif selon n'importe quelle revendication précédente, dans lequel l'élément de suivi (30, 140) présente une bobine (33, 157) au niveau d'une pointe distale.

10. Dispositif selon n'importe quelle revendication précédente, comprenant en outre un film thermorétractable (35, 153) sur une région de connexion des éléments de suivi et de coupe (30, 140', 20, 120', 130').

11. Dispositif selon n'importe quelle revendication précédente, dans lequel l'élément de coupe (20, 120, 130) est un fil métallique (51, 70, 170, 175, 180, 181, 182, 184, 188) et l'élément de suivi (30, 140) est un fil métallique (55).

12. Dispositif selon n'importe quelle revendication précédente, dans lequel l'élément de coupe (20, 120, 130) présente une coupe transversale sensiblement circulaire dans une région proximale (56) et une coupe transversale sensiblement triangulaire dans une région distale (51).

13. Combinaison d'un dispositif selon n'importe quelle revendication précédente avec un cathéter (100) présentant une lumière, dans laquelle l'au moins un élément de coupe comporte une pluralité d'éléments de coupe (120, 130), la pluralité d'éléments de coupe étant connectée au niveau d'une partie distale (142) à l'élément de suivi (140) et pouvant être insérée en tant que tout à travers la lumière du cathéter (100).

14. Combinaison d'un dispositif selon l'une quelconque des revendications 1 à 12 avec un cathéter (60, 100) présentant une lumière (62, 102) et une partie extensible (64, 104), dans lequel l'élément de coupe (20, 120, 120', 130, 130') présente une surface de coupe configurée pour traiter la lésion afin d'agrandir le passage dans la lumière corporelle quand il est déplacé par la partie extensible du cathéter pour élargir l'espace entre l'élément de coupe et l'élément de suivi (30, 140).

15. Combinaison selon la revendication 14, dans lequel la partie extensible (64, 104) comprend un ballon gonflable.
